# EUROPEAN PATENT APPLICATION

(11) **EP 4 744 671 A1**
(43) Date of publication of application: **20.05.2026**
(21) Application number: 24838825.8
(22) Date of filing: 10.07.2024
(51) Int. Cl.: A61K 31/765, A61L 27/18, A61L 27/54, A61L 27/56, A61L 27/58, A61K 47/34, A61P 19/00

(54) **USE OF POLYLACTIC ACID AND COPOLYMER THEREOF FOR PROMOTING GROWTH OF BONE TISSUE AND CARTILAGE TISSUE**

(30) Priority: 12.07.2023 CN 202310853821
(71) Applicant: Changchun Sinobiomaterials Co., Ltd., Changchun, Jilin 130000 (CN)
(72) Inventor: WANG, Jinyue, Changchun, Jilin 130000 (CN); ZHUANG, Xiuli, Changchun, Jilin 130000 (CN); ZHANG, Tianhui, Changchun, Jilin 130000 (CN)
(74) Representative: Colombo, Stefano Paolo
(86) International application number: PCT/CN2024/104667
(87) International publication number: WO 2025/011573

(57) **Abstract**

The use of polylactic acid and a copolymer thereof or a microsphere thereof in the preparation of a drug for promoting the growth of bone tissue and cartilage tissue. The molecular weight of the polylactic acid and the copolymer thereof is 400 Da to 300 kDa. It is found for the first time that the polylactic acid and the copolymer thereof or the microsphere thereof have an effect of promoting the growth of bone tissue and cartilage tissue, thereby providing a new way for promoting the growth of bone tissue and cartilage tissue.

## Description

### CROSS-REFERENCE TO RELATED APPLICATIONS

The present application claims priority to Chinese Patent Application No. 202310853821.9, filed on July 12, 2023, entitled "USE OF POLYLACTIC ACID AND COPOLYMER THEREOF FOR PROMOTING GROWTH OF CARTILAGE TISSUE", the entire content of which are incorporated herein by reference.

### TECHNICAL FIELD

The present invention relates to the technical field of polymer medical materials, in particular to use of polylactic acid and copolymer thereof for promoting growth of bone tissue and cartilage tissue.

### BACKGROUND

Polylactic acid (PLA) has the molecular formula -[OCH(CH₃)CO]- and is mainly prepared by lactic acid polymerization or lactide ring-opening polymerization. Its structural formula is

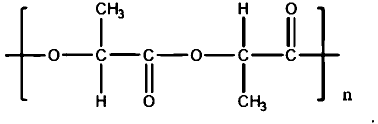

PLA and a copolymer thereof not only possess excellent mechanical strength and chemical stability, but also exhibit good biocompatibility and biodegradability. In recent years, a great deal of research has been conducted both domestically and internationally on use of PLA and the copolymer thereof in biomedicine. PLA and the copolymer thereof has been widely used in surgical sutures, bone repair materials, controlled-release drug systems, and tissue functional scaffolds (such as artificial bone and artificial skin).

Among them, poly-L-lactic acid (trade name SculptraTM, PLLA) contains biodegradable and biocompatible synthetic polymer, i.e., poly-L-lactic acid microparticles. In 2004, PLLA was approved by the U.S. FDA for filling sunken and sagging skin areas caused by facial lipoatrophy in AIDS patients. In 2009, the US FDA officially approved PLLA fillers for improving nasolabial folds.

However, there are no reports in the existing technology regarding whether polylactic acid and a copolymer thereof or a microsphere thereof can be used as a polymeric drug to promote the growth of bone tissue and cartilage tissue.

### SUMMARY

Aiming at the shortcomings existing in the prior art, the present invention provides use of polylactic acid and a copolymer thereof or a microsphere thereof in the preparation of a drug for promoting the growth of bone tissue and cartilage tissue, and provides a new way for promoting the growth of bone tissue and cartilage tissue.

The present invention provides use of polylactic acid and a copolymer thereof, a polylactic acid microsphere and a polylactic acid copolymer microsphere in the preparation of a drug for promoting the growth of bone tissue and cartilage tissue, wherein the molecular weight of the polylactic acid and the copolymer thereof is 400 Da - 300 kDa; preferably, the polylactic acid and the copolymer thereof have a molecular weight of 5000 Da - 100 kDa; For example, 5000 Da - 20 kDa, and 15000 Da.

According to an embodiment of the present invention, the polylactic acid comprise poly-L-lactic acid, poly-D-lactic acid, and racemic polylactic acid; preferably poly-L-lactic acid.

According to an embodiment of the present invention, the polylactic acid copolymer comprises one or more of polylactic acid-glycolic acid copolymer, polylactic acid-polyethylene glycol copolymer, polyethylene glycol-polylactic acid-glycolic acid copolymer, polylactic acid-chitosan copolymer, and lactide-caprolactone copolymer; preferably, the polylactic acid copolymer is polylactic acid-glycolic acid copolymer.

According to an embodiment of the present invention, the method for preparing a polylactic acid microsphere and a polylactic acid copolymer microsphere comprises the following steps:
1) mixing polylactic acid or the polylactic acid copolymer with a solvent to obtain a polylactic acid solution or a polylactic acid copolymer solution;
   preferably, the solvent is any one of dichloromethane, chloroform, ethyl acetate, acetone or toluene, or a mixture of any two or more thereof;
2) mixing the polylactic acid solution or the polylactic acid copolymer solution with a polyvinyl alcohol aqueous solution, performing high-speed emulsification, stirring the resulting emulsion to remove the solvent, and performing freeze-drying to obtain the polylactic acid microsphere.

According to an embodiment of the present invention, in step 1), the mass-volume ratio of polylactic acid or the polylactic acid copolymer to the solvent is 1 g : 5 mL - 1 g : 30 mL, preferably 1 g : 10 mL - 1 g : 20 mL, for example 1 g: 15 mL;
and/or, in step 2), a mass concentration of the polyvinyl alcohol aqueous solution is 0.05% - 5.0%, preferably 0.1% - 2.0%, for example 0.5%;
and/or, in step 2), a volume ratio of the polylactic acid solution or the polylactic acid copolymer solution to the polyvinyl alcohol aqueous solution is 1:5 - 1:30, preferably 1:10 - 1:20, for example 1:13, 1:15;
and/or, in step 2), a speed of the high-speed emulsification is 1000 rpm/min-5000 rpm/min, for example, 3000 rpm/min;
and/or, in step 2), the emulsification time is 5 - 20 min, for example, 10 min.

According to an embodiment of the present invention, when the drug is administrated, the mass concentration of polylactic acid and the copolymer thereof, the polylactic acid microsphere, and the polylactic acid copolymer microsphere is 1% - 60%, preferably 5% - 50%, for example 10%, 20%, 30%, and 40%.

For example, polylactic acid and the copolymer thereof, the polylactic acid microsphere, or the polylactic acid copolymer microsphere is prepared as a solution with the above-mentioned mass concentration.

According to an embodiment of the present invention, the drug may optionally further comprises an excipient;
preferably, the excipient comprises at least one of a stabilizer, a filler, a binder, a surfactant or a lubricant;
preferably, the surfactant is selected from one or more of polyethylene glycol, sodium dodecyl sulfonate, Tween, and Span;
preferably, the stabilizer is selected from one or both of carboxymethyl cellulose and mannitol;
preferably, the filler is selected from one or more of lactose, mannitol, cyclodextrin, and sorbitol;
preferably, the binder is selected from one or more of hydroxypropyl cellulose, methyl cellulose, sodium hyaluronate, collagen, and polyvinyl pyrrolidone;
preferably, the lubricant is selected from one or more of magnesium stearate, calcium stearate and stearic acid.

According to an embodiment of the present invention, the drug is optionally used in combination with other active ingredients, wherein the other active ingredients have the effect of promoting the growth of bone tissue and cartilage tissue;
preferably, the other active ingredients are selected from one or more of collagen, gelatin, bone gla protein (BGP), bonemorphogenetic protein (BMP), basic fibroblast growth factor (bFGF), insulin like growth factor (IGF), transforming growth factor-β (TGF-β), platelet derived growth factor (PDGF), concentrated growth factor (CGF)-containing fibrin, platelet-rich fibrin (PRF), etc.

According to an embodiment of the present invention, an administration route of the drug comprises, but is not limited to, intravenous injection, in situ injection, intramuscular injection, subcutaneous injection, oral administration, topical application, etc.

According to an embodiment of the present invention, a dosage form of the drug is an injection, a tablet, a granule, a capsule, an oral liquid, an ointment, etc.; preferably an injection.

Compared with the prior art, the present invention has the following beneficial effects:
the present invention found for the first time that polylactic acid and a copolymer thereof, a polylactic acid microsphere and a polylactic acid copolymer microsphere have an effect of promoting the growth of bone tissue and cartilage tissue, thereby providing a new way for promoting the growth of bone tissue and cartilage tissue.

### BRIEF DESCRIPTION OF THE DRAWINGS

Drawings illustrated here are used for providing a further understanding of the invention and form part of the application. Exemplary examples and illustrations thereof are intended to explain the invention and not to improperly limit the invention. In the drawings:
FIG. 1 shows a scanning electron microscope (SEM) image of the poly-L-lactic acid (PLLA) microsphere in Example 1.
FIG. 2 shows a scanning electron microscope (SEM) image of the polylactic acid-glycolic acid copolymer (PLGA) microsphere in Example 2.
FIG. 3 is a graph showing the results of the effect of PLLA microsphere solution on the proliferation behavior of osteoblasts.
FIG. 4 is a graph showing the results of the effect of PLGA microsphere solution on the proliferation behavior of chondrocytes.
FIG. 5 shows the results of the test of the relative expression levels of cartilage-related genes after in vitro culture of PLGA microsphere solution-cell complex.
FIG. 6 shows the immunohistochemical results of the effect of PLGA microsphere solution on cartilage tissue growth.

### DETAILED DESCRIPTION

The technical solution of the present invention will be further described in detail below in conjunction with specific examples. It should be understood that the following examples are only intended to exemplify and explain the present invention and should not be construed as limiting the scope of protection of the present invention. All technologies realized based on the above contents of the present invention are included in the scope that the present invention intends to protect.

Unless otherwise indicated, the raw materials and reagents used in the following examples are commercially available or may be prepared by known methods.

### Example 1 Preparation and characterization of poly-L-lactic Acid microsphere

9 g of poly-L-lactic acid (PLLA) (molecular weight 15000 g/mol) was dissolved in 135 mL of dichloromethane. The polymer solution was then added to 1800 mL of a polyvinyl alcohol aqueous solution at a mass concentration of 0.5%. After emulsification at 3000 rpm/min for 10 minutes, the mixture was stirred at 500 rpm for 3 hours to remove the dichloromethane. After freeze-drying, the poly-L-lactic acid microspheres were obtained.

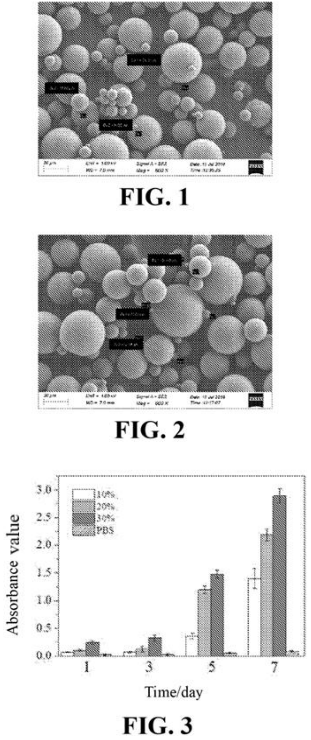

The prepared poly-L-lactic acid microspheres were observed using a scanning electron microscope and their particle size was calculated. The SEM image of the poly-L-lactic acid microspheres is shown in FIG. 1. The particle size range of the microspheres is 3.72~33.12 µm.

### Example 2 Preparation and characterization of polylactic acid-glycolic acid copolymer microspheres

9 g of polylactic acid-glycolic acid copolymer (PLGA) (molecular weight 15000 g/mol) was dissolved in 135 mL of dichloromethane. The polymer solution was then added to 1800 mL of a polyvinyl alcohol aqueous solution at a mass concentration of 0.5%. After emulsification at 3000 rpm/min for 10 minutes, the mixture was stirred at 500 rpm for 3 hours to remove the dichloromethane. After freeze-drying, the polylactic acid-glycolic acid copolymer microspheres were obtained.

The prepared polylactic acid-glycolic acid copolymer microspheres were observed using a scanning electron microscope and their particle size was calculated. The SEM image of the PLGA microspheres is shown in FIG. 2. The particle size range of the microspheres is 2.94~36.92 µm.

### Example 3 Experiment on the effect of poly-L-lactic acid (PLLA) microsphere solution on osteoblast proliferation

The effect of the PLLA microsphere solution in Example 1 on the proliferation of mouse embryonic osteoblasts MC3T3 was tested using the MTT assay.

PBS buffer was used as a blank control group, and PLLA microsphere solutions with mass concentrations of 10%, 20%, and 30% were prepared as experimental groups using PBS buffer. First, the samples of each of the above-mentioned groups were added to a 96-well plate, with 100µL per well, and 5 replicate wells was set up for each sample. Then, 100µL of MC3T3 cell suspension at a concentration of 1×10⁴/mL was added to each sample well and the plate was placed in a cell culture incubator. After incubating in the dark for 1, 3, 5, and 7 days, 20µL of MTT solution was added to each well. Following 4 hours of dark incubation, the supernatant in each well was removed, and then 150µL of dimethyl sulfoxide was used for dissolution until a clear solution was obtained. The reading at 570 nm was measured using a microplate reader, and the final results of the effects of PLLA microsphere solutions at different concentrations on the proliferation behavior of MC3T3 cells were calculated.

The results are shown in FIG. 3. Compared with the blank control group, PLLA microsphere solutions at different concentrations all promoted the proliferation of osteoblasts. Moreover, as the concentration of PLLA microspheres increased, the number of proliferating osteoblasts gradually increased. It can be seen that the PLLA microsphere solution has a significant effect on promoting osteoblast proliferation.

### Example 4 Experiment on the effect of poly-L-lactic acid PLLA microsphere solution on bone tissue growth

### 1. Establishment of osteoporosis model:

Forty 3-month-old non-pregnant female SD rats weighing approximately 200 g were selected, and bilateral ovariectomy was performed. The rats were then reared for 3 months postoperatively.

### 2. Implantation of microspheres and detection of in vivo osteogenesis:

### (1) Specimen preparation

The rats were anesthetized and placed in a prone position on the surgical panel. The bilateral femur greater trochanters were incised and exposed, and a bone defect with a diameter of 2.5 mm and a depth of 3 mm was created using an electric drill from the greater trochanter toward the lesser trochanter. 40 ovariectomized rats were randomly divided into 5 groups, with 8 rats in each group. The groups were as follows: control group (sham-operated), buffer solution group (PBS buffer was injected into the femoral intertrochanteric bone defect area), low-concentration PLLA microsphere group (microspheres at a concentration of 5%/PBS buffer), medium-concentration PLLA microsphere group (microspheres at a concentration of 10%/PBS buffer), and high-concentration PLLA microsphere group (microsphere at a concentration of 20%/PBS buffer). 30µL of the aforementioned samples was injected into both defect sites of the rats, followed by layered suturing of the muscle and skin. At 3 months post-operation, 8 rats from each group were euthanized. The intact femurs were extracted, cleaned of soft tissue, wrapped in saline-soaked gauze, and stored at -20°C. The PLLA microspheres used were prepared as described in Example 1.

### (2) Determination of bone mineral density in the intertrochanteric region of the femur

The femoral specimen was scanned using an X-ray bone densitometer to determine the bone mineral content (BMC) and bone mineral density (BMD) in the intertrochanteric region of the femur. As shown in Table 1, at 3 months post-operation, the bone mineral content and bone mineral density in the microsphere sample group were not lower than those in the control group and buffer solution group. Moreover, with increasing microsphere concentration, the values of bone mineral content and bone mineral density gradually increased (the differences in bone mineral content and bone mineral density in the intertrochanteric region of the femur were statistically significant).

**Table 1 Comparison of bone mineral content and bone mineral density in the intertrochanteric region of the femur at 3 months post-operation among different sample groups (n=8)**

| | Control group | Buffer group | 5% microsphere group | 10% microsphere group | 20% microsphere group |
|---|---|---|---|---|---|
| BMC (g) | 0.037±0.002 | 0.029±0.002 | 0.043±0.003 | 0.052±0.004 | 0.058±0.002 |
| BMD (g/cm²) | 0.251±0.011 | 0.201±0.010 | 0.292+0.031 | 0.338±0.019 | 0.387±0.012 |

### (3) Observation of trabecular bone structure in the intertrochanteric region of the femur by Micro CT

The femur was transected below the lesser trochanter, and the proximal portion was placed along the long axis in a sample container. Scanning was performed at 80 kV with a resolution of 6.8µm to determine the bone morphometric parameters in the intertrochanteric region of the femur. Among them: 1) percentage of trabecular area (%Tb.Ar): the percentage of trabecular area relative to the total bone tissue area, reflecting the amount of bone mass; 2) trabecular thickness (Tb.Th): used to describe the structural morphology of trabeculae, with a certain quantity, greater thickness indicates more bone mass; 3) trabecular spacing (Tb.Sp): used to describe the average distance between trabeculae, greater spacing indicates larger gaps between trabeculae and more porous bone structure.

As can be seen from the data in Table 2, at 3 months post-operation, the trabecular thickness values in the microsphere sample groups were significantly higher than those in the control group and buffer solution group. Moreover, as the microsphere concentration increased, the trabecular thickness gradually increased (the differences between groups were all statistically significant). Furthermore, the percentage of trabecular area in the 20% microsphere sample group was significantly higher than that in the control group and buffer solution group, but there was no statistically significant difference compared with the 5% and 10% microsphere groups. At 3 months post-operation, the trabecular spacing values in all microsphere sample groups were lower than those in the control group and buffer solution group. Moreover, as the microsphere concentration increased, the trabecular spacing gradually decreased (the differences between groups were all statistically significant).

**Table 2 Comparison of trabecular histomorphology in the intertrochanteric region of the femur at 3 months post-operation among different sample groups (n=8)**

| | Control group | Buffer group | 5% microsphere group | 10% microsphere group | 20% microsphere group |
|---|---|---|---|---|---|
| Tb.Th (µm) | 100.65±6.29 | 85.57±12.01 | 216.32+39.62 | 269.92±75.66 | 371.91±39.65 |
| %Tb.Ar (%) | 5.23±0.30 | 4.81±1.12 | 6.17±0.19 | 6.29±0.92 | 6.51±0.27 |
| Tb.Sp (µm) | 4757.18±555.91 | 4671.25±587.49 | 3712.15+151.87 | 1769.06±113.91 | 1560.02±56.99 |

### Example 5 Experiment on effect of polylactic acid-glycolic acid copolymer PLGA microsphere solution on chondrocytes

### 1. Chondrocyte extraction and culture

Chondrocytes were harvested from auricular cartilage tissues of three-week-old New Zealand white rabbits and passaged once after approximately one week of culture. The third-generation chondrocytes were used for microsphere material seeding in the present invention.

### 2. MTT assay for the effect of PLGA microsphere solution on chondrocyte proliferation

PBS buffer was used as a blank control group, and PLGA microsphere solutions with mass concentrations of 10%, 20%, and 40% were prepared as experimental groups using PBS buffer. First, the samples of each of the above-mentioned groups were added to a 96-well plate, with 100µL of microsphere solution per well, and 5 replicate wells was set up for each sample. Then, 100µL of cell suspension at a concentration of 1×10⁴/mL was added to each sample well and the plate was placed in a cell culture incubator. After incubating in the dark for 2, 4, 6, and 8 days, 20µL of MTT solution was added to each well. Following 4 hours of dark incubation, the supernatant in each well was removed, and then 150µL of dimethyl sulfoxide was used for dissolution until a clear solution was obtained. The reading at 570 nm was measured using a microplate reader, and the final proliferation results of chondrocytes on microsphere samples with different concentrations were calculated. The polylactic acid-glycolic acid copolymer microspheres were prepared as described in Example 2.

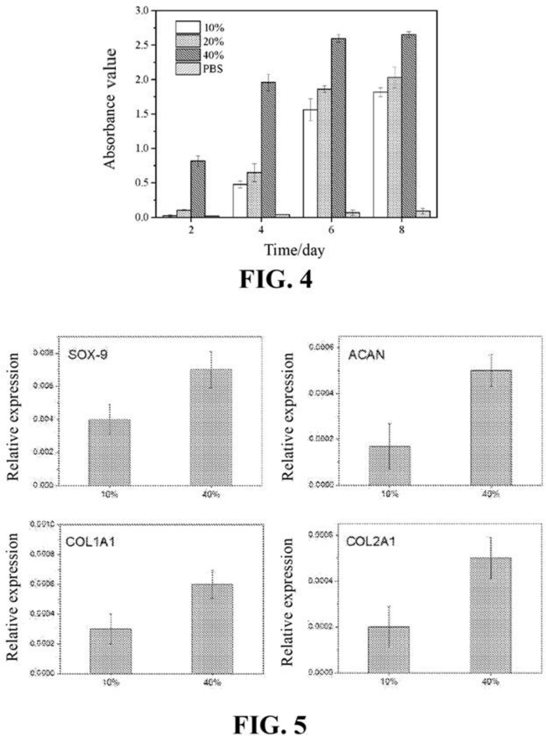

The test results are shown in FIG. 4. Compared with the blank control group, PLGA microsphere solutions at different concentrations all promoted the proliferation of chondrocytes. Furthermore, as the culture time and microsphere concentration increased, the number of chondrocytes in the sample groups gradually increased, indicating that PLGA microspheres can promote chondrocyte proliferation.

### Example 6 Effect of polylactic acid-glycolic acid copolymer PLGA microsphere solution on cartilage growth

### 1. Isolation of chondrocytes

Under sterile conditions, a porcine auricular cartilage tissue measuring approximately 2cm×2cm was excised. After removing the skin and subcutaneous tissue under sterile conditions, the perichondrium was excised and the cartilage was cut into approximately 1mm³ pieces, which were digested with 0.25% trypsin for 40 minutes, followed by the addition of 5 volumes of 0.2% collagenase for continued digestion for 16-24 hours. After filtering the cells through a cell strainer, the precipitate was collected by repeated centrifugation and finally placed in a constant-temperature incubator for culture to obtain P0 generation porcine auricular chondrocytes.

### 2. Mixing of chondrocytes with PLGA microspheres

PLGA microsphere solutions with mass concentrations of 10% and 40% were prepared as experimental groups using PBS buffer. P2 generation porcine auricular chondrocytes were selected to prepare a cell suspension with a density of 1×10⁸/mL. An appropriate amount of cell suspension, PLGA microsphere solution, and culture medium were added together into a 6-well plate and further cultured in a constant temperature incubator. The specific groupings are shown in Table 3.

**Table 3 Specific component information of Group A and Group B**

| | **Name** | **Component** |
|---|---|---|
| Group A | Low concentration microsphere group | 100µL of cell suspension + 100µL of 10% PLGA microsphere solution + 2 mL of culture medium |
| Group B | High concentration microsphere group | 100µL of cell suspension + 100µL of 40% PLGA microsphere solution + 2 mL of culture medium |

### 3. Real-time PCR

After 4 weeks of in vitro culture of cell microspheres, the expression of cartilage-related genes was detected by real-time PCR, and the expression levels of each group relative to the internal reference gene GAPDH were statistically analyzed.
1) Reaction system: 0.3µl of each of upstream and downstream primers, 5µl of SYBR, 0.4 µl of a template, and 4 µl of ddH₂O.
2) Reaction program: 95°C for 1 min, 95°C for 5 s, 60°C for 1 min, with 40 cycles.
3) Quantitative analysis was performed using GAPDH in each group as an internal reference.
4) Primer sequences are shown in Table 4.

**Table 4 Real-time PCR primer sequences**

| Gene name | Upstream and downstream primers | Primer sequence (5'-3') |
|---|---|---|
| SOX9 | Upstream | 5-ACTACACAGACCACCAGAACTCCG-3 SEQ ID NO:1 |
| | Downstream | 5-TGAAGGTGGAGTAGAGCACAGAGC-3 SEQ ID NO:2 |
| ACAN | Upstream | 5-TGGGAAAGAAGACTTGGCTGG-3 SEQ ID NO:3 |
| | Downstream | 5-CTCCACCAATGTAGTATCCACGA-3 SEQ ID NO:4 |
| COL2A1 | Upstream | 5-TGGGCAGAGGTATAATGATAAGGA-3 SEQ ID NO:5 |
| | Downstream | 5-CTTCACAGATTATGTCGTCGCAG-3 SEQ ID NO:6 |
| COL1A1 | Upstream | 5-TGCTCCCCAGTTGTCTTATG-3 SEQ ID NO:7 |
| | Downstream | 5-ATCATCTCCGTTCTTGCCAG-3 SEQ ID NO:8 |

FIG. 5 shows the test results of the relative expression levels of cartilage-related genes after in vitro culture of microsphere-chondrocyte complex. The results indicate that PLGA microspheres promote chondrogenesis. Compared with the low-concentration microsphere samples, the high-concentration microsphere samples exhibited significantly increased expression levels of cartilage-related genes, with statistically significant differences.

### Example 7 Effect of polylactic acid-glycolic acid copolymer PLGA microsphere solution on cartilage growth

### 1. In vivo injection of microsphere solution

3 healthy Bama miniature pigs, male, 2 months old, weighing 8-10 kg, were selected. After anesthesia and disinfection, subcutaneous cavities were created in the pigs following sterile procedures. PBS buffer solution was used as the control group and PLGA microsphere solutions with mass concentrations of 20% and 60% prepared with PBS buffer solution were used as the sample groups. 1 mL of the aforementioned control group and sample groups were injected subcutaneously into both sides of the pigs' backs, respectively. The injection sites were then sutured and marked. At the 2nd week post-injection, the materials were extracted and placed in formalin for histological staining.

### 2. Histological staining

### (1) Preparation of tissue sections

1) Trimming the 10% formaldehyde-fixed tissues into appropriately sized tissue blocks.
2) Gradient ethanol dehydration: 70% ethanol for 30 minutes, 80% ethanol for 30 minutes, 90% ethanol for 30 minutes, 95% ethanol I for 30 minutes, 95% ethanol II for 30 minutes, absolute ethanol I for 40 minutes, absolute ethanol II for 40 minutes, absolute ethanol : xylene (1 : 1) for 40 minutes, xylene for 8 minutes twice.
3) After removal from xylene, immediately transferring the tissue blocks to paraffin : xylene (1 : 1) for 40 minutes, then to soft paraffin with a melting point of 50-52°C for 40 minutes, and finally to hard paraffin with a melting point of 60-62°C for 40 minutes.
4) Embedding the tissues in hard paraffin and leaving the embedded tissues at room temperature overnight.
5) Sectioning the tissues with a microtome at a thickness of 5-8µm.
6) Floating the sections on distilled water at 45°C.
7) Baking the sections at 50°C for 1.5-2 hours, then storing for later use.

### 3. Immunohistochemical staining of type II collagen

1) After thawing the frozen sections at room temperature for 1 hour, fixing the thawed sections with 4% paraformaldehyde for 15 minutes, followed by washing with PBS 3 times, 3 minutes each.
2) Performing antigen retrieval according to the pepsin antigen retrieval kit protocol, adding hydrogen peroxide-methanol solution dropwise and incubating the mixture at room temperature for 10 minutes, followed by washing with PBS 3 times, 3 minutes each.
3) Incubation with pepsin for 30 minutes, followed by washing with PBS 3 times, 3 minutes each.
4) Following the steps of the immunohistochemistry kit to use 3% hydrogen peroxide solution for endogenous enzyme blocking at room temperature.
5) Washing with PBS 3 times, 3 minutes each.
6) Blocking nonspecific antigens with 10% goat serum for 10 minutes.
7) Diluting the primary antibody with PBS according to the instructions, discarding the goat serum, then adding the primary antibody dropwise and incubating the mixture overnight at 4°C.
8) Allowing the mixture to equilibrate at room temperature for 1-2 hours.
9) Discarding the primary antibody, followed by washing with PBS 3 times, 3 minutes each.
10) Adding the secondary antibody, followed by incubation at 37°C for 30 minutes.
11) Washing with PBS 3 times, 3 minutes each.
12) Preparing DAB chromogenic solution according to the instructions, adding the chromogenic solution under the microscope, and terminating the chromogenic reaction when positive results appeared.
13) Hematoxylin staining for 2 minutes, followed by washing with water.
14) Soaking in tap water for 10 minutes.
15) Dehydration with absolute ethanol for 5 minutes.
16) Xylene I clearing treatment for 5 minutes, xylene II clearing treatment for 5 minutes, followed by mounting with neutral resin.

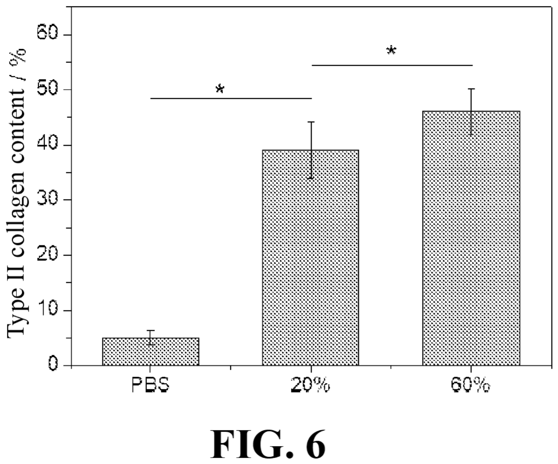

The immunohistochemical results are shown in FIG. 6. The results demonstrate that after 2 weeks of in vivo injection, compared with the PBS buffer solution group, the PLGA microsphere solution sample groups all exhibited significant positive expression of type II collagen. Statistical analysis revealed that the type II collagen expression level in the high-concentration microsphere solution was significantly higher than that in the low-concentration microsphere solution. The results indicate that the PLGA microsphere solution can promote cartilage growth.

Finally, it should be noted that the above examples are only used to illustrate the technical solutions of the present invention and not to limit them. Although the present invention has been described in detail with reference to the preferred examples, those of ordinary skill in the art should understand that the technical solutions of the present invention can be modified or equivalently replaced without departing from the purpose and scope of the technical solutions of the present invention, and all of them shall be covered by the scope of the claims of the present invention.

## Claims

1. Use of polylactic acid and a copolymer thereof, a polylactic acid microsphere and a polylactic acid copolymer microsphere in the preparation of a drug for promoting the growth of bone tissue and cartilage tissue, wherein the molecular weight of the polylactic acid and the copolymer thereof is 400 Da to 300 kDa;
preferably, the molecular weight of the polylactic acid and the copolymer thereof is 5000 Da-100 kDa; for example, 5000 Da-20 kDa, or 15000 Da.

2. The use according to claim 1, wherein
the polylactic acid comprise poly-L-lactic acid, poly-D-lactic acid, and racemic polylactic acid; preferably poly-L-lactic acid.

3. The use according to claim 1, wherein the polylactic acid copolymer comprises one or more of polylactic acid-glycolic acid copolymer, polylactic acid-polyethylene glycol copolymer, polyethylene glycol-polylactic acid-glycolic acid copolymer, polylactic acid-chitosan copolymer, and lactide-caprolactone copolymer;
preferably, the polylactic acid copolymer is polylactic acid-glycolic acid copolymer.

4. The use according to any one of claims 1-3, wherein a method for preparing the polylactic acid microsphere and the polylactic acid copolymer microsphere comprises the following steps:
1) mixing polylactic acid or the polylactic acid copolymer with a solvent to obtain a polylactic acid solution or a polylactic acid copolymer solution;
preferably, the solvent is any one of dichloromethane, chloroform, ethyl acetate, acetone or toluene, or a mixture of any two or more thereof;
2) mixing the polylactic acid solution or the polylactic acid copolymer solution with a polyvinyl alcohol aqueous solution, performing high-speed emulsification, stirring the resulting emulsion to remove the solvent, and performing freeze-drying to obtain the polylactic acid microsphere.

5. The use according to claim 4, wherein
in step 1), a mass-volume ratio of polylactic acid or the polylactic acid copolymer to the solvent is 1 g : 5 mL - 1 g : 30 mL, preferably 1 g : 10 mL - 1 g : 20 mL, for example 1 g : 15 mL;
and/or, in step 2), a mass concentration of the polyvinyl alcohol aqueous solution is 0.05% - 5.0%, preferably 0.1% - 2.0%, for example 0.5%;
and/or, in step 2), a volume ratio of the polylactic acid solution or the polylactic acid copolymer solution to the polyvinyl alcohol aqueous solution is 1:5 - 1:30, preferably 1:10 - 1:20, for example 1:13, 1:15;
and/or, in step 2), a speed of the high-speed emulsification is 1000 rpm/min-5000 rpm/min, for example, 3000 rpm/min;
and/or, in step 2), the emulsification time is 5 - 20 min, for example, 10 min.

6. The use according to any one of claims 1-5, wherein when the drug is administrated, a mass concentration of polylactic acid and the copolymer thereof, the polylactic acid microsphere, and the polylactic acid copolymer microsphere is 1% - 80%, preferably 5% - 50%, for example 10%, 20%, 30%, and 40%.

7. The use according to any one of claims 1-6, wherein the drug optionally further comprises an excipient;
preferably, the excipient comprises at least one of a stabilizer, a filler, a binder, a surfactant or a lubricant;
preferably, the surfactant is selected from one or more of polyethylene glycol, sodium dodecyl sulfonate, Tween, and Span;
preferably, the stabilizer is selected from one or both of carboxymethyl cellulose and mannitol;
preferably, the filler is selected from one or more of lactose, mannitol, cyclodextrin, and sorbitol;
preferably, the binder is selected from one or more of hydroxypropyl cellulose, methyl cellulose, sodium hyaluronate, collagen, and polyvinyl pyrrolidone;
preferably, the lubricant is selected from one or more of magnesium stearate, calcium stearate and stearic acid.

8. The use according to any one of claims 1-7, wherein the drug is optionally used in combination with other active ingredients, wherein the other active ingredients have the effect of promoting the growth of bone tissue and cartilage tissue;
preferably, the other active ingredients are selected from one or more of collagen, gelatin, bone gla protein (BGP), bonemorphogenetic protein (BMP), basic fibroblast growth factor (bFGF), insulin like growth factor (IGF), transforming growth factor-β (TGF-β), platelet derived growth factor (PDGF), concentrated growth factor (CGF)-containing fibrin, platelet-rich fibrin (PRF), etc.

9. The use according to any one of claims 1-8, wherein an administration route of the drug comprises, but is not limited to, intravenous injection, in situ injection, intramuscular injection, subcutaneous injection, oral administration, topical application, etc.

10. The use according to any one of claims 1-8, wherein a dosage form of the drug is an injection, a tablet, a granule, a capsule, an oral liquid, an ointment, etc.; preferably an injection.
